(19) Europäisches Patentamt
European Patent Office
Office européen des brevets

(11) **EP 3 159 688 A1**

(12) **EUROPEAN PATENT APPLICATION**

(43) Date of publication:
**26.04.2017 Bulletin 2017/17**

(51) Int Cl.:
***G01N 33/22*** *(2006.01)*

(21) Application number: **16195143.9**

(22) Date of filing: **21.10.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.10.2015 US 201562245604 P**
**14.06.2016 US 201615181921**

(71) Applicant: **AIR PRODUCTS AND CHEMICALS, INC.**
**Allentown, PA 18195-1501 (US)**

(72) Inventors:
• **HERB, Blaine Edward**
**New Triplo, PA 18066 (US)**
• **MACCONNELL, Matthew H**
**Orefield, PA 18069 (US)**
• **PENG, Xiang-Dong**
**Orefield, PA 18069 (US)**

(74) Representative: **Beck Greener**
**Fulwood House**
**12 Fulwood Place**
**London WC1V 6HR (GB)**

(54) **METHOD AND APPARATUS FOR DETERMINING THE HEATING VALUE OF FUEL GASES**

(57) Method and apparatus for determining a heating value of a sample hydrocarbon-containing mixture having hydrocarbons and non-hydrocarbons as mixture components. The sample is reacted with an excess amount of oxidant gas in a reaction chamber to form a product gas containing residual oxygen. The molecular weight of the sample is measured and the residual oxygen concentration of the product gas is measured. The heating value is calculated from the measurements of the molecular weight and the residual oxygen concentration. Pure component hydrocarbon gases may be used to calibrate the system.

FIG. 1

**Description**

CROSS-REFERENCE TO RELATED APPLICATIONS

This application claims priority to provisional application U.S. Ser. No. 62/245,604, titled "Method and Apparatus for Determining Heating Value," filed 23 October 2015, the contents of which are hereby incorporated by reference.

BACKGROUND

The present invention relates to a method and apparatus for determining the heating value of hydrocarbon-containing mixtures.

Industry desires to determine the heating value of fuel gases. The heating value of a fuel gas may be important for determining the value and/or cost of the fuel gas. The heating value of the fuel may also be important for controlling the heat input to various types of furnaces.

Some prior art methods and apparatuses for determining the heating value of hydrocarbon-containing mixtures require the use of one or more calibration gases where the calibration gases are certified mixtures of multiple components. Industry desires a method and apparatus where pure component calibration gases can be used.

Industry desires a method and apparatus for determining the heating value of hydrocarbon-containing mixtures with improved accuracy.

Industry desires a method and apparatus for determining the heating value of hydrocarbon-containing mixtures for mixtures containing non-hydrocarbon components, such as nitrogen and carbon dioxide.

Industry desires methods to determine heating value that are "universally applicable" so the need to perform a substantial amount of work to customize the method to each individual application is eliminated.

Industry desires methods that apply broadly over extremely wide ranges of concentrations of each component in the fuel gas mixture.

Industry desires methods that provide high accuracy over wide ranges of concentrations of each component in the fuel gas mixture.

Industry desires methods that allow for the fuel mixtures to include any of a large number of possible components.

Industry desires methods which do not rely on the use of calibration gas mixtures and are not influenced by the inherent uncertainty of the composition of the calibration gas mixtures.

BRIEF SUMMARY

There are several aspects of the invention as outlined below. In the following, specific aspects of the invention are outlined below. The reference numbers and expressions set in parentheses are referring to an example embodiment explained further below with reference to the figures. The reference numbers and expressions are, however, only illustrative and do not limit the aspect to any specific component or feature of the example embodiment. The aspects can be formulated as claims in which the reference numbers and expressions set in parentheses are omitted or replaced by others as appropriate.

Aspect 1. An apparatus for determining a heating value of a sample hydrocarbon-containing mixture having hydrocarbons and non-hydrocarbons as mixture components, the apparatus comprising:

a reaction chamber configured to selectively receive the sample hydrocarbon-containing mixture and an oxidant gas and discharge a product gas having a residual $O_2$ concentration where the product gas is formed from the sample hydrocarbon-containing mixture and the oxidant gas;

a first sensor configured to acquire a measured value relatable to the residual $O_2$ concentration in the product gas and for generating an electronic signal in response thereto;

a second sensor configured to acquire a measured value relatable to the molecular weight of the sample hydrocarbon-containing mixture and for generating an electronic signal in response thereto; and

a computing device operatively connected to the first sensor and the second sensor to receive the electronic signals from the first sensor and the second sensor, wherein the computing device is configured to calculate the heating value of the sample hydrocarbon-containing mixture from a combustion oxygen requirement index and the measured value relatable to molecular weight, wherein the combustion oxygen requirement index is determined using a correlation of the combustion oxygen requirement index as a function of the residual $O_2$ concentration and using the measured value relatable to the residual $O_2$ concentration in the product gas as input to the correlation.

Aspect 2. The apparatus of aspect 1 further comprising:

a third sensor configured to acquire a measured value relatable to a hydrogen concentration of the sample hydrocarbon-containing mixture and for generating an electronic signal in response thereto;

wherein the computing device is also operatively connected to the third sensor to receive the electronic signals from the third sensor, wherein the computing device is configured to calculate the heating value of the sample hydrocarbon-containing mixture from the measured value relatable to the hydrogen concentration of the sample hydrocarbon-containing mixture in addition to the combustion oxygen requirement index and the measured value relatable to the molecular weight.

Aspect 3. The apparatus of aspect 1 or aspect 2 wherein the computing device is configured to calculate the heating value of the sample hydrocarbon-containing mixture using a mathematical relationship derived from one or more model equations where mixture properties are determined from additive contributions from at least a first group of components and a second group of components.

Aspect 4. The apparatus of any one of aspects 1 to 3 further comprising:

a source of a calibration gas, the calibration gas having a concentration of a single hydrocarbon component greater than 99 mole % or greater than 99.9 mole %, wherein the reaction chamber is configured to selectively receive the calibration gas from the source of the calibration gas and to receive the oxidant gas, the reaction chamber configured to receive the calibration gas severally (i.e. each by itself) from the sample hydrocarbon-containing mixture, the reaction chamber configured to discharge a product gas formed from the calibration gas, the product gas formed from the calibration gas having a residual $O_2$ concentration;

wherein the first sensor is configured to acquire a measured value relatable to the residual $O_2$ concentration in the product gas formed from the calibration gas and for generating an electronic signal in response thereto;

wherein the second sensor is configured to acquire a measured value relatable to the molecular weight of the calibration gas and for generating an electronic signal in response thereto; and

wherein the computing device is configured to calibrate the correlation of the combustion oxygen requirement index as a function of the residual $O_2$ concentration using the measured value relatable to the residual $O_2$ concentration in the product gas formed from the calibration gas and the measured value relatable to the molecular weight of the calibration gas.

Aspect 5. The apparatus of aspect 4 further comprising:

a source of a second calibration gas, the second calibration gas having a concentration of a single hydrocarbon component greater than 99 mole % or greater than 99.9 mole %, where the hydrocarbon component in the second calibration gas is different than the hydrocarbon component in the calibration gas;

wherein the reaction chamber is configured to selectively receive the second calibration gas from the source of the second calibration gas and to receive the oxidant gas, the reaction chamber configured to receive the second calibration gas severally from the sample hydrocarbon-containing mixture and severally from the calibration gas, the reaction chamber configured to discharge a product gas formed from the second calibration gas, the product gas formed from the second calibration gas having a residual $O_2$ concentration;

wherein the first sensor is configured to acquire a measured value relatable to the residual $O_2$ concentration in the product gas formed from the second calibration gas and for generating an electronic signal in response thereto;

wherein the second sensor is configured to acquire a measured value relatable to the molecular weight of the second calibration gas and for generating an electronic signal in response thereto; and

wherein the computing device is configured to calibrate the correlation of the combustion oxygen requirement index as a function of the residual $O_2$ concentration using the measured value relatable to the residual $O_2$ concentration in the product gas formed from the second calibration gas and the measured value relatable to the molecular weight of the second calibration gas.

Aspect 6. The apparatus of any one of aspects 1 to 5 further comprising:

a catalyst in the reaction chamber.

Aspect 7. The apparatus of any one of the preceding aspects further comprising:

a first orifice operatively arranged to receive the oxidant gas prior to the reaction chamber receiving the oxidant gas; and

a second orifice operatively arranged to receive the sample hydrocarbon-containing mixture prior to the reaction chamber receiving the sample hydrocarbon-containing mixture.

Aspect 8. The apparatus of aspect 7 wherein the second orifice is operatively arranged to receive the calibration gas prior to the reaction chamber receiving the calibration gas.

Aspect 9. The apparatus of aspect 8 wherein the second orifice is operatively arranged to receive the second calibration gas prior to the reaction chamber receiving the second calibration gas.

Aspect 10. A method for determining a heating value of a sample hydrocarbon-containing mixture having hydrocarbons and non-hydrocarbons as mixture components, the method comprising:

(a) reacting the sample hydrocarbon-containing mixture with an oxidant gas, the sample hydrocarbon-containing mixture and the oxidant gas provided in a ratio to form a product gas having a residual $O_2$ concentration;

(b) acquiring a measured value relatable to the residual $O_2$ concentration in the product gas;

(c) acquiring a measured value relatable to molecular weight of the sample hydrocarbon-containing mixture; and

(d) determining a heating value of the sample hydrocarbon-containing mixture from a combustion oxygen requirement index and the measured value relatable to molecular weight, wherein the combustion oxygen requirement index is determined using a correlation of the combustion oxygen requirement index as a function of the residual $O_2$ concentration and the measured value relatable to the residual $O_2$ concentration.

Aspect 11. The method of aspect 10 further comprising:

acquiring a measured value relatable to a hydrogen concentration of the sample hydrocarbon-containing mixture;

wherein the heating value of the sample hydrocarbon-containing mixture is also determined from the measured value relatable to the hydrogen concentration of the sample hydrocarbon-containing mixture.

Aspect 12. The method of aspect 10 or aspect 11 wherein the heating value of the sample hydrocarbon-containing mixture is determined using a mathematical relationship derived from one or more model equations where mixture properties are determined from additive contributions from at least a first group of components and a second group of components.

Aspect 13. The method of any one of aspects 10 to 12 wherein prior to steps (a) - (d), the method further comprises:

reacting a calibration gas with the oxidant gas, the calibration gas having a concentration of a single hydrocarbon component greater than 99 mole % or greater than 99.9 mole %, the calibration gas and the oxidant gas provided in a ratio to form a product gas having residual $O_2$ concentration;

acquiring a measured value relatable to the residual $O_2$ concentration in the product gas formed from the calibration gas;

acquiring a measured value relatable to molecular weight of the calibration gas; and calibrating the correlation of the combustion oxygen requirement index as a function of the residual $O_2$ concentration using the measured value relatable to the residual $O_2$ concentration in the product gas formed from the calibration gas and the measured value relatable to the molecular weight of the calibration gas.

Aspect 14. The method of aspect 13 wherein prior to steps (a) - (d), the method further comprises:

reacting a second calibration gas with the oxidant gas, the second calibration gas having a concentration of a single hydrocarbon component greater than 99 mole % or greater than 99.9 mole % where the hydrocarbon component in the second calibration gas is different than the hydrocarbon component in the calibration gas, the second calibration gas and the oxidant gas provided in a ratio to form a product gas having residual $O_2$ concentration;

acquiring a measured value relatable to the residual $O_2$ concentration in the product gas formed from the second calibration gas;

acquiring a measured value relatable to molecular weight of the second calibration gas;

calibrating the correlation of the combustion oxygen requirement index as a function of the residual $O_2$ concentration using the measured value relatable to the residual $O_2$ concentration in the product gas formed from the second calibration gas and the measured value relatable to the molecular weight of the second calibration gas.

Aspect 15. The method of aspect 10 wherein the sample hydrocarbon-containing mixture is reacted with the oxidant gas in the presence of a catalyst.
Aspect 16. The method of any one of aspects 10 to 15 further comprising:

calculating a carbon content value of the sample hydrocarbon-containing mixture using the combustion oxygen requirement index of the sample hydrocarbon-containing mixture and the measured value relatable to the molecular weight of the sample hydrocarbon-containing mixture in a carbon content correlation.

Aspect 17. The method of any one aspects 10 to 16 further comprising:

calculating a carbon content value of the sample hydrocarbon-containing mixture using the heating value of the sample hydrocarbon-containing mixture and the measured value relatable to the molecular weight of the sample hydrocarbon-containing mixture in a carbon content correlation.

Aspect 18. The method of any one of aspects 10 to 17 further comprising:

passing the oxidant through a first orifice under choked flow conditions and into a reaction chamber; and

passing the sample hydrocarbon-containing mixture through a second orifice under choked flow conditions and into the reaction chamber for reacting the sample hydrocarbon-containing mixture with the oxidant gas in the reaction chamber, said passing of the sample hydrocarbon-containing mixture through the second orifice contemporaneous with said passing of the oxidant through the first orifice.

Aspect 19. The method of aspect 18 further comprising
passing the calibration gas through the second orifice under choked flow conditions and into the reaction chamber for reacting the calibration gas with the oxidant gas in the reaction chamber, said calibration gas passed through the second orifice severally from the sample hydrocarbon-containing mixture, said passing of the calibration gas through the second orifice contemporaneous with said passing of the oxidant through the first orifice.
Aspect 20. The method of aspect 19 further comprising
passing the second calibration gas through the second orifice under choked flow conditions and into the reaction chamber for reacting the second calibration gas with the oxidant gas in the reaction chamber, said second calibration gas passed through the second orifice severally from the sample hydrocarbon-containing mixture and severally from the calibration gas, said passing of the second calibration gas through the second orifice contemporaneous with said passing of the oxidant through the first orifice.
Aspect 21. An apparatus for determining a heating value of a sample hydrocarbon-containing mixture having hydrocarbons and non-hydrocarbons as mixture components, the apparatus comprising:

a reaction chamber configured to selectively receive the sample hydrocarbon-containing mixture and an oxidant gas, and to discharge a product gas having residual $O_2$ concentration where the product gas is formed from the sample hydrocarbon-containing mixture and the oxidant gas;

a first sensor configured to acquire a measured value relatable to the residual $O_2$ concentration in the product gas and for generating an electronic signal in response thereto;

a second sensor configured to acquire a measured value relatable to the molecular weight of the sample hydrocarbon-containing mixture and for generating an electronic signal in response thereto;

a third sensor configured to acquire a measured value relatable to a hydrogen concentration of the sample hydrocarbon-containing mixture and for generating an electronic signal in response thereto;

a computing device operatively connected to the first sensor, second sensor, and the third sensor to receive the electronic signals from the first sensor, the second sensor, and the third sensor, wherein the computing device is configured to calculate the heating value of the sample hydrocarbon-containing mixture from the measured value relatable to the residual $O_2$ concentration in the product gas, the measured value relatable to the molecular weight of the sample hydrocarbon-containing mixture, and the measured value relatable to a hydrogen concentration of the sample hydrocarbon-containing mixture.

Aspect 22. The apparatus of aspect 21 further comprising:

a source of a calibration gas, the calibration gas having a concentration of a single hydrocarbon component greater than 99 mole % or greater than 99.9 mole %,

wherein the reaction chamber is configured to selectively receive the calibration gas from the source of the calibration gas and to receive the oxidant gas, the reaction chamber configured to receive the calibration gas severally (i.e. each by itself) from the sample hydrocarbon-containing mixture, the reaction chamber configured to discharge a product gas formed from the calibration gas, the product gas formed from the calibration gas having a residual $O_2$ concentration;

wherein the first sensor is configured to acquire a measured value relatable to the residual $O_2$ concentration in the product gas formed from the calibration gas and for generating an electronic signal in response thereto;

wherein the second sensor is configured to acquire a measured value relatable to the molecular weight of the calibration gas and for generating an electronic signal in response thereto; and

wherein the computing device is configured to calculate the heating value of the sample hydrocarbon-containing mixture from the residual $O_2$ concentration in the product gas formed from the calibration gas and the measured value relatable to the molecular weight of the calibration gas in addition to the measured value relatable to the residual $O_2$ concentration in the product gas formed from the sample hydrocarbon-containing mixture, the measured value relatable to the molecular weight of the sample hydrocarbon-containing mixture, and the measured value relatable to a hydrogen concentration of the sample hydrocarbon-containing mixture.

Aspect 23. The apparatus of aspect 22 further comprising:

a source of a second calibration gas, the second calibration gas having a concentration of a single hydrocarbon component greater than 99 mole % or greater than 99.9 mole %, where the hydrocarbon component in the second calibration gas is different than the hydrocarbon component in the calibration gas;

wherein the reaction chamber is configured to selectively receive the second calibration gas from the source of the second calibration gas and to receive the oxidant gas, the reaction chamber configured to receive the second calibration gas severally from the sample hydrocarbon-containing mixture and severally from the calibration gas, the reaction chamber configured to discharge a product gas formed from the second calibration gas, the product gas formed from the second calibration gas having a residual $O_2$ concentration;

wherein the first sensor is configured to acquire a measured value relatable to the residual $O_2$ concentration in the product gas formed from the second calibration gas and for generating an electronic signal in response thereto;

wherein the second sensor is configured to acquire a measured value relatable to the molecular weight of the second calibration gas and for generating an electronic signal in response thereto; and

wherein the computing device is configured to calculate the heating value of the sample hydrocarbon-containing mixture from the residual $O_2$ concentration in the product gas formed from the second calibration gas and the measured value relatable to the molecular weight of the second calibration gas in addition to the residual $O_2$ con-

centration in the product gas formed from the calibration gas and the measured value relatable to the molecular weight of the calibration gas, the measured value relatable to the residual $O_2$ concentration in the product gas formed from the sample hydrocarbon-containing mixture, the measured value relatable to the molecular weight of the sample hydrocarbon-containing mixture, and the measured value relatable to a hydrogen concentration of the sample hydrocarbon-containing mixture.

Aspect 24. The apparatus of any one of aspects 21 to 23 wherein the computing device is configured to calculate the heating value of the sample hydrocarbon-containing mixture from a combustion oxygen requirement index wherein the combustion oxygen requirement index is determined using a correlation of the combustion requirement oxygen index as a function of the residual $O_2$ concentration and using the measured value relatable to the residual $O_2$ concentration in the product gas as input to the correlation.

Aspect 25. The apparatus of aspect 24 including aspect 22 wherein the correlation of the combustion oxygen requirement index as a function of the residual $O_2$ concentration is calibrated using the measured value relatable to the residual $O_2$ concentration in the combustion product gas formed from the calibration gas and the measured value relatable to the molecular weight of the calibration gas.

Aspect 26. The apparatus of aspect 24 including aspect 23 wherein the correlation of the combustion oxygen requirement index as a function of the residual $O_2$ concentration is calibrated using the measured value relatable to the residual $O_2$ concentration in the combustion product gas formed from the second calibration gas and the measured value relatable to the molecular weight of the second calibration gas.

Aspect 27. The apparatus of any one of aspects 21 to 26 wherein the computing device is configured to calculate the heating value of the sample hydrocarbon-containing mixture using a mathematical relationship derived from one or more model equations where mixture properties are determined from additive contributions from at least a first group of components and a second group of components.

Aspect 28. The apparatus of any one of aspects 21 to 27 further comprising:

a catalyst in the reaction chamber.

Aspect 29. The apparatus of any one of aspects 21 to 28 further comprising:

a first orifice operatively arranged to receive the oxidant gas prior to the reaction chamber receiving the oxidant gas; and

a second orifice operatively arranged to receive the sample hydrocarbon-containing mixture prior to the reaction chamber receiving the sample hydrocarbon-containing mixture.

Aspect 30. The apparatus of aspect 29 wherein the second orifice is operatively arranged to receive the calibration gas prior to the reaction chamber receiving the calibration gas.

Aspect 31. The apparatus of aspect 30 wherein the second orifice is operatively arranged to receive the second calibration gas prior to the reaction chamber receiving the second calibration gas.

Aspect 32. A method for determining a heating value of a sample hydrocarbon-containing mixture having hydrocarbons and non-hydrocarbons as mixture components, the method comprising:

(a) reacting the sample hydrocarbon-containing mixture with an oxidant gas, the sample hydrocarbon-containing mixture and the oxidant gas provided in a ratio to form a product gas having a residual $O_2$ concentration;

(b) acquiring a measured value relatable to the residual $O_2$ concentration in the product gas;

(c) acquiring a measured value relatable to molecular weight of the sample hydrocarbon-containing mixture;

(d) acquiring a measured value relatable to a hydrogen concentration of the sample hydrocarbon-containing mixture; and

(e) determining a heating value of the sample hydrocarbon-containing mixture from the measured value relatable to the residual $O_2$ concentration in the product gas, the measured value relatable to molecular weight of the sample hydrocarbon-containing mixture, and the measured value relatable to a hydrogen concentration of the sample hydrocarbon-containing mixture.

Aspect 33. The method of aspect 32 wherein prior to steps (a) - (e), the method further comprises:

reacting a calibration gas with the oxidant gas (severally from the sample gas), the calibration gas having a concentration of a single hydrocarbon component greater than 99 mole % or greater than 99.9 mole %, the calibration gas and the oxidant gas provided in a ratio to form a product gas having residual $O_2$ concentration;

acquiring a measured value relatable to the residual $O_2$ concentration in the product gas formed from the calibration gas; and

acquiring a measured value relatable to molecular weight of the calibration gas;

wherein the heating value of the sample hydrocarbon-containing mixture is determined from the measured value relatable to the residual $O_2$ concentration in the product gas formed from the calibration gas, and the measured value relatable to molecular weight of the calibration gas in addition to the measured value relatable to the residual $O_2$ concentration in the product gas formed from the sample hydrocarbon-containing mixture, the measured value relatable to molecular weight of the sample hydrocarbon-containing mixture, and the measured value relatable to a hydrogen concentration of the sample hydrocarbon-containing mixture.

Aspect 34. The method of aspect 33 wherein prior to steps (a) - (e), the method further comprises:

reacting a second calibration gas with the oxidant gas, the second calibration gas having a concentration of a single hydrocarbon component greater than 99 mole % or greater than 99.9 mole % where the hydrocarbon component in the second calibration gas is different than the hydrocarbon component in the calibration gas, the second calibration gas and the oxidant gas provided in a ratio to form a product gas having residual $O_2$ concentration;

acquiring a measured value relatable to the residual $O_2$ concentration in the product gas formed from the second calibration gas;

acquiring a measured value relatable to molecular weight of the second calibration gas;

wherein the heating value of the sample hydrocarbon-containing mixture is determined from the measured value relatable to the residual $O_2$ concentration in the product gas formed from the second calibration gas, and the measured value relatable to molecular weight of the second calibration gas in addition to the measured value relatable to the residual $O_2$ concentration in the product gas formed from the calibration gas, the measured value relatable to the molecular weight of the calibration gas, the measured value relatable to the residual $O_2$ concentration in the product gas formed from the sample hydrocarbon-containing mixture, the measured value relatable to molecular weight of the sample hydrocarbon-containing mixture, and the measured value relatable to a hydrogen concentration of the sample hydrocarbon-containing mixture.

Aspect 35. The method of any one of aspects 32 to 34 wherein in the step of determining the heating value, a combustion oxygen requirement index is determined using a correlation of the combustion oxygen requirement index as a function of the residual $O_2$ concentration and the measured value relatable to the residual $O_2$ concentration of the sample hydrocarbon-containing mixture, and the heating value is calculated from the combustion oxygen requirement index and the measured value relatable to molecular weight of the sample hydrocarbon-containing mixture.

Aspect 36. The method of aspect 35 including aspect 33 wherein the correlation of the combustion oxygen requirement index as a function of the residual $O_2$ concentration is calibrated using the measured value relatable to the residual $O_2$ concentration in the combustion product gas formed from the calibration gas and the measured value relatable to the molecular weight of the calibration gas.

Aspect 37. The method of aspect 35 including aspect 34 wherein the correlation of the combustion oxygen requirement index as a function of the residual $O_2$ concentration is calibrated using the measured value relatable to the residual $O_2$ concentration in the combustion product gas formed from the second calibration gas and the measured value relatable to the molecular weight of the second calibration gas.

Aspect 38. The method of any one of aspects 32 to 37 wherein the heating value of the sample hydrocarbon-containing mixture is determined using a mathematical relationship derived from one or more model equations where mixture properties are determined from additive contributions from at least a first group of components and a second group of components.

Aspect 39. The method of aspect 32 to 38 wherein the sample hydrocarbon-containing mixture is reacted with the oxidant gas in the presence of a catalyst.

Aspect 40. The method of any one of aspects 32 to 39 further comprising:

passing the oxidant through a first orifice under choked flow conditions and into a reaction chamber; and

passing the sample hydrocarbon-containing mixture through a second orifice under choked flow conditions and into the reaction chamber for reacting the sample hydrocarbon-containing mixture with the oxidant gas in the reaction chamber, said passing of the sample hydrocarbon-containing mixture through the second orifice contemporaneous with said passing of the oxidant through the first orifice.

Aspect 41. The method of aspect 40 further comprising
passing the calibration gas through the second orifice under choked flow conditions and into the reaction chamber for reacting the calibration gas with the oxidant gas in the reaction chamber, said calibration gas passed through the second orifice severally from the sample hydrocarbon-containing mixture, said passing of the calibration gas through the second orifice contemporaneous with said passing of the oxidant through the first orifice.

Aspect 42. The method of aspect 41 further comprising
passing the second calibration gas through the second orifice under choked flow conditions and into the reaction chamber for reacting the second calibration gas with the oxidant gas in the reaction chamber, said second calibration gas passed through the second orifice severally from the sample hydrocarbon-containing mixture and severally from the calibration gas, said passing of the second calibration gas through the second orifice contemporaneous with said passing of the oxidant through the first orifice.

Aspect 43. The method of any one of aspects 32 to 42 further comprising:

calculating a carbon content value of the sample hydrocarbon-containing mixture using the combustion oxygen requirement index of the sample hydrocarbon-containing mixture and the measured value relatable to the molecular weight of the sample hydrocarbon-containing mixture in a carbon content correlation.

Aspect 44. The method of any one of aspects 32 to 43 further comprising:

calculating a carbon content value of the sample hydrocarbon-containing mixture using the heating value of the sample hydrocarbon-containing mixture and the measured value relatable to the molecular weight of the sample hydrocarbon-containing mixture in a carbon content correlation.

Aspect 45. An apparatus for determining a heating value of a sample hydrocarbon-containing mixture having hydrocarbons and non-hydrocarbons as mixture components, the apparatus comprising:

a source of a calibration gas, the calibration gas having a concentration of a single hydrocarbon component greater than 99 mole % or greater than 99.9 mole %,

a reaction chamber configured to selectively receive the sample hydrocarbon-containing mixture and an oxidant gas, and to discharge a product gas having residual $O_2$ concentration where the product gas is formed from the sample hydrocarbon-containing mixture and the oxidant gas, wherein the reaction chamber is configured to selectively receive the calibration gas from the source of the calibration gas and to receive the oxidant gas with the calibration gas, the reaction chamber configured to receive the calibration gas severally (i.e. each by itself) from the sample hydrocarbon-containing mixture, the reaction chamber configured to discharge a product gas formed from the calibration gas, the product gas formed from the calibration gas having a residual $O_2$ concentration;

a first sensor configured to acquire a measured value relatable to the residual $O_2$ concentration in the product gas formed from the sample hydrocarbon-containing mixture and for generating an electronic signal in response thereto, and the first sensor is configured to acquire a measured value relatable to the residual $O_2$ concentration in the product gas formed from the calibration gas and for generating an electronic signal in response thereto;

a second sensor configured to acquire a measured value relatable to the molecular weight of the sample hydrocarbon-containing mixture and for generating an electronic signal in response thereto, and the second sensor is configured to acquire a measured value relatable to the molecular weight of the calibration gas and for generating an electronic signal in response thereto;

a computing device operatively connected to the first sensor and the second sensor to receive the electronic signals from the first sensor and the second sensor, wherein the computing device is configured to calculate the heating value of the sample hydrocarbon-containing mixture from the residual $O_2$ concentration in the product gas formed from the calibration gas, the measured value relatable to the molecular weight of the calibration gas, the measured value relatable to the residual $O_2$ concentration in the product gas formed from the sample hydrocarbon-containing mixture, and the measured value relatable to the molecular weight of the sample hydrocarbon-containing mixture.

Aspect 46. The apparatus of aspect 45 further comprising:

a source of a second calibration gas, the second calibration gas having a concentration of a single hydrocarbon component greater than 99 mole % or greater than 99.9 mole %, where the hydrocarbon component in the second calibration gas is different than the hydrocarbon component in the calibration gas;

wherein the reaction chamber is configured to selectively receive the second calibration gas from the source of the second calibration gas and to receive the oxidant gas, the reaction chamber configured to receive the second calibration gas severally from the sample hydrocarbon-containing mixture and severally from the calibration gas, the reaction chamber configured to discharge a product gas formed from the second calibration gas, the product gas formed from the second calibration gas having a residual $O_2$ concentration;

wherein the first sensor is configured to acquire a measured value relatable to the residual $O_2$ concentration in the product gas formed from the second calibration gas and for generating an electronic signal in response thereto;

wherein the second sensor is configured to acquire a measured value relatable to the molecular weight of the second calibration gas and for generating an electronic signal in response thereto; and

wherein the computing device is configured to calculate the heating value of the sample hydrocarbon-containing mixture from the residual $O_2$ concentration in the product gas formed from the second calibration gas and the measured value relatable to the molecular weight of the second calibration gas in addition to the residual $O_2$ concentration in the product gas formed from the calibration gas, the measured value relatable to the molecular weight of the calibration gas, the measured value relatable to the residual $O_2$ concentration in the product gas formed from the sample hydrocarbon-containing mixture, and the measured value relatable to the molecular weight of the sample hydrocarbon-containing mixture.

Aspect 47. The apparatus of aspect 45 or aspect 46 further comprising:

a third sensor configured to acquire a measured value relatable to a hydrogen concentration of the sample hydrocarbon-containing mixture and for generating an electronic signal in response thereto;

wherein the computing device is also operatively connected to the third sensor to receive the electronic signals from the third sensor, wherein the computing device is configured to calculate the heating value of the sample hydrocarbon-containing mixture from the measured value relatable to the hydrogen concentration of the sample hydrocarbon-containing mixture.

Aspect 48. The apparatus of any one of aspects 45 to 47 wherein the computing device is configured to calculate the heating value of the sample hydrocarbon-containing mixture from a combustion oxygen requirement index wherein the combustion oxygen requirement index is determined using a correlation of the combustion oxygen requirement index as a function of the residual $O_2$ concentration and using the measured value relatable to the residual $O_2$ concentration in the product gas as input to the correlation.

Aspect 49. The apparatus of aspect 48 wherein the correlation of the combustion oxygen requirement index as a function of the residual $O_2$ concentration is calibrated using the measured value relatable to the residual $O_2$ concentration in the combustion product gas formed from the calibration gas and the measured value relatable to the molecular weight of the calibration gas.

Aspect 50. The apparatus of aspect 48 including aspect 46 wherein the correlation of the combustion oxygen requirement index as a function of the residual $O_2$ concentration is calibrated using the measured value relatable to the residual $O_2$ concentration in the combustion product gas formed from the second calibration gas and the measured value relatable to the molecular weight of the second calibration gas.

Aspect 51. The apparatus of any one of aspects 45 to 50 wherein the computing device is configured to calculate the heating value of the sample hydrocarbon-containing mixture using a mathematical relationship derived from one or more model equations where mixture properties are determined from additive contributions from at least a first group of components and a second group of components.

Aspect 52. The apparatus of any one of aspects 45 to 51 further comprising:

a catalyst in the reaction chamber.

Aspect 53. The apparatus of any one of aspects 45 to 52 further comprising:

a first orifice operatively arranged to receive the oxidant gas prior to the reaction chamber receiving the oxidant gas; and

a second orifice operatively arranged to receive the sample hydrocarbon-containing mixture prior to the reaction chamber receiving the sample hydrocarbon-containing mixture.

Aspect 54. The apparatus of aspect 53 wherein the second orifice is operatively arranged to receive the calibration gas prior to the reaction chamber receiving the calibration gas.
Aspect 53. The apparatus of aspect 54 wherein the second orifice is operatively arranged to receive the second calibration gas prior to the reaction chamber receiving the second calibration gas.
Aspect 54. A method for determining a heating value of a sample hydrocarbon-containing mixture having hydrocarbons and non-hydrocarbons as mixture components, the method comprising:

(a) reacting a calibration gas with the oxidant gas (severally from the sample gas), the calibration gas having a concentration of a single hydrocarbon component greater than 99 mole % or greater than 99.9 mole %, the calibration gas and the oxidant gas provided in a ratio to form a product gas having residual $O_2$ concentration;

(b) acquiring a measured value relatable to the residual $O_2$ concentration in the product gas formed from the calibration gas; and

(c) acquiring a measured value relatable to molecular weight of the calibration gas;

(d) reacting the sample hydrocarbon-containing mixture with an oxidant gas, the sample hydrocarbon-containing mixture and the oxidant gas provided in a ratio to form a product gas having a residual $O_2$ concentration;

(e) acquiring a measured value relatable to the residual $O_2$ concentration in the product gas;

(f) acquiring a measured value relatable to molecular weight of the sample hydrocarbon-containing mixture; and

(g) determining a heating value of the sample hydrocarbon-containing mixture from the measured value relatable to the residual $O_2$ concentration in the product gas formed from the calibration gas, and the measured value relatable to molecular weight of the calibration gas, the measured value relatable to the residual $O_2$ concentration in the product gas formed from the sample hydrocarbon-containing mixture, and the measured value relatable to molecular weight of the sample hydrocarbon-containing mixture.

Aspect 55. The method of aspect 54 wherein prior to steps (d) - (g), the method further comprises:

reacting a second calibration gas with the oxidant gas, the second calibration gas having a concentration of a single hydrocarbon component greater than 99 mole % or greater than 99.9 mole % where the hydrocarbon component in the second calibration gas is different than the hydrocarbon component in the calibration gas, the second calibration gas and the oxidant gas provided in a ratio to form a product gas having residual $O_2$ concentration;

acquiring a measured value relatable to the residual $O_2$ concentration in the product gas formed from the second calibration gas;

acquiring a measured value relatable to molecular weight of the second calibration gas;

wherein the heating value of the sample hydrocarbon-containing mixture is determined from the measured value relatable to the residual $O_2$ concentration in the product gas formed from the second calibration gas, and the measured value relatable to molecular weight of the second calibration gas in addition to the measured value relatable to the residual $O_2$ concentration in the product gas formed from the calibration gas, the measured value relatable to the molecular weight of the calibration gas, the measured value relatable to the residual $O_2$ concentration in the product gas formed from the sample hydrocarbon-containing mixture, the measured value relatable to molecular weight of the sample hydrocarbon-containing mixture, and the measured value relatable to a hydrogen concentration of the sample hydrocarbon-containing mixture.

Aspect 56. The method of aspect 54 or aspect 55 further comprising:

acquiring a measured value relatable to a hydrogen concentration of the sample hydrocarbon-containing mixture;

wherein the heating value of the sample hydrocarbon-containing mixture is also determined from the measured value relatable to the hydrogen concentration of the sample hydrocarbon-containing mixture.

Aspect 57. The method of any one of aspects 54 to 56 wherein in the step of determining the heating value, a combustion oxygen requirement index is determined using a correlation of the combustion oxygen requirement index as a function of the residual $O_2$ concentration and the measured value relatable to the residual $O_2$ concentration of the sample hydrocarbon-containing mixture, and the heating value is calculated from the combustion oxygen requirement index and the measured value relatable to molecular weight of the sample hydrocarbon-containing mixture.

Aspect 58. The method of aspect 57 wherein the correlation of the combustion oxygen requirement index as a function of the residual $O_2$ concentration is calibrated using the measured value relatable to the residual $O_2$ concentration in the combustion product gas formed from the calibration gas and the measured value relatable to the molecular weight of the calibration gas.

Aspect 59. The method of aspect 57 including aspect 55 wherein the correlation of the combustion oxygen requirement index as a function of the residual $O_2$ concentration is calibrated using the measured value relatable to the residual $O_2$ concentration in the combustion product gas formed from the second calibration gas and the measured value relatable to the molecular weight of the second calibration gas.

Aspect 60. The method of any one of aspects 54 to 59 wherein the heating value of the sample hydrocarbon-containing mixture is determined using a mathematical relationship derived from one or more model equations where mixture properties are determined from additive contributions from at least a first group of components and a second group of components.

Aspect 61. The method of aspect 54 to 60 wherein the sample hydrocarbon-containing mixture is reacted with the oxidant gas in the presence of a catalyst.

Aspect 62. The method of any one of the preceding aspects further comprising:

passing the oxidant through a first orifice under choked flow conditions and into a reaction chamber; and

passing the sample hydrocarbon-containing mixture through a second orifice under choked flow conditions and into the reaction chamber for reacting the sample hydrocarbon-containing mixture with the oxidant gas in the reaction chamber, said passing of the sample hydrocarbon-containing mixture through the second orifice contemporaneous with said passing of the oxidant through the first orifice.

Aspect 63. The method of aspect 62 further comprising
passing the calibration gas through the second orifice under choked flow conditions and into the reaction chamber for reacting the calibration gas with the oxidant gas in the reaction chamber, said calibration gas passed through the second orifice severally from the sample hydrocarbon-containing mixture, said passing of the calibration gas through the second orifice contemporaneous with said passing of the oxidant through the first orifice.

Aspect 64. The method of aspect 63 further comprising
passing the second calibration gas through the second orifice under choked flow conditions and into the reaction chamber for reacting the second calibration gas with the oxidant gas in the reaction chamber, said second calibration gas passed through the second orifice severally from the sample hydrocarbon-containing mixture and severally from the calibration gas, said passing of the second calibration gas through the second orifice contemporaneous with said passing of the oxidant through the first orifice.

Aspect 65. The method of any one of aspects 54 to 64 further comprising:

calculating a carbon content value of the sample hydrocarbon-containing mixture using the combustion oxygen requirement index of the sample hydrocarbon-containing mixture and the measured value relatable to the molecular weight of the sample hydrocarbon-containing mixture in a carbon content correlation.

Aspect 66. The method of any one of aspects 54 to 65 further comprising:

calculating a carbon content value of the sample hydrocarbon-containing mixture using the heating value of the sample hydrocarbon-containing mixture and the measured value relatable to the molecular weight of the sample hydrocarbon-containing mixture in a carbon content correlation.

BRIEF DESCRIPTION OF SEVERAL VIEWS OF THE DRAWINGS

FIG. 1 is a plot of COR as a function of higher heating value for alkanes, alkenes, CO and $H_2$.

FIG. 2 is a plot of molecular weight as a function of higher heating value for alkanes, alkenes, CO and $H_2$.

FIG. 3 is a plot of COR as a function of residual $O_2$ concentration.

FIG. 4 is a plot of *CORI* as a function of residual $O_2$ concentration.

FIG. 5 is a calibration curve of CARI as a function of residual $O_2$ concentration for a calibration mixture of methane and ethane.

DETAILED DESCRIPTION OF THE PREFERRED EMBODIMENTS

The ensuing detailed description provides preferred exemplary embodiments only, and is not intended to limit the scope, applicability, or configuration of the invention. Rather, the ensuing detailed description of the preferred exemplary embodiments will provide those skilled in the art with an enabling description for implementing the preferred exemplary embodiments of the invention, it being understood that various changes may be made in the function and arrangement of elements without departing from scope of the invention as defined by the claims.

The articles "a" and "an" as used herein mean one or more when applied to any feature in embodiments of the present invention described in the specification and claims. The use of "a" and "an" does not limit the meaning to a single feature unless such a limit is specifically stated. The article "the" preceding singular or plural nouns or noun phrases denotes a particular specified feature or particular specified features and may have a singular or plural connotation depending upon the context in which it is used.

The adjective "any" means one, some, or all indiscriminately of whatever quantity.

The term "and/or" placed between a first entity and a second entity includes any of the meanings of (1) only the first entity, (2) only the second entity, and (3) the first entity and the second entity. The term "and/or" placed between the last two entities of a list of 3 or more entities means at least one of the entities in the list including any specific combination of entities in this list. For example, "A, B and/or C" has the same meaning as "A and/or B and/or C" and comprises the following combinations of A, B and C: (1) only A, (2) only B, (3) only C, (4) A and B and not C, (5) A and C and not B, (6) B and C and not A, and (7) A and B and C.

The present invention relates to a method and apparatus for determining a heating value of a sample hydrocarbon-containing mixture having hydrocarbons and non-hydrocarbons as mixture components. The heating value that is determined may be a lower heating value or a higher heating value. Lower heating value and higher heating value are common terms used in the field of combustion. A lower heating value, also called net heating value, is the gross heating value minus the latent heat of vaporization of the water vapor formed by the combustion of the hydrogen in the fuel. Higher heating value, also called gross heating value, is the total heat obtained from combustion of a specified amount of fuel and its stoichiometrically correct amount of oxidant (e.g. air), both being at 60°F when combustion starts and the combustion products being cooled to 60°F before the heat release is measured.

The present approach for determining heating value of the hydrocarbon-containing mixture is a destructive approach, meaning that the sample is consumed in order to determine the heating value. Therefore a small sample of the hydrocarbon-containing mixture is diverted from a given process and the sample is used to determine the heating value.

The method comprises reacting the sample hydrocarbon-containing mixture with an oxidant gas. The oxidant gas may be any suitable oxidant gas containing oxygen. The oxidant gas may most conveniently be air. The oxidant gas may be industrial grade oxygen, i.e. essentially pure oxygen having an oxygen concentration greater than 99 mole % or greater than 99.9 mole %. The oxidant gas may be an oxidant gas having an oxygen concentration between that of air and industrial grade oxygen.

The sample hydrocarbon-containing mixture and the oxidant gas are provided in a ratio to form a product gas having a residual $O_2$ concentration. The sample hydrocarbon-containing mixture and oxidant gas may be reacted in the presence of a catalyst. Any suitable catalyst known in the art may be used.

The apparatus comprises a reaction chamber. The reaction chamber is configured to receive the sample hydrocarbon-containing mixture and the oxidant gas.

The reaction chamber may contain a catalyst to support complete reaction of the hydrocarbons in the sample hydrocarbon-containing mixture.

The reaction chamber is configured to discharge a product gas formed from the sample hydrocarbon-containing mixture. The sample hydrocarbon-containing mixture and oxidant gas are combined in a ratio to form a product gas having a residual $O_2$ concentration, i.e. an excess amount of oxygen is provided.

The pressure and temperature of sample hydrocarbon-containing mixture and oxidant gas may be equalized (i.e. made the same as each other) using pressure regulators and heat exchangers.

The method may comprise passing the oxidant through a first orifice under choked flow conditions and into a reaction chamber.

The apparatus may comprise a first orifice. The first orifice may be operatively arranged so that the oxidant gas passes first through the first orifice and then to the reaction chamber.

The first orifice may be any type of orifice, for example, an orifice plate or a valve. The first orifice is sized such that, for the pressure and temperature of the oxidant gas, choked flow occurs. This is so that a fixed constant flow of oxidant gas may be achieved.

The method may comprise passing the sample hydrocarbon-containing mixture through a second orifice under choked flow conditions and into the reaction chamber for reacting the sample hydrocarbon-containing mixture with the oxidant gas in the reaction chamber. The sample hydrocarbon-containing mixture may be passed through the second orifice contemporaneously with the passing of the oxidant through the first orifice

The apparatus may comprise a second orifice. The second orifice may be operatively arranged so that the sample hydrocarbon-containing mixture passes first through the second orifice and then to the reaction chamber.

The second orifice may be any type of orifice, for example, an orifice plate or a valve. The second orifice is sized such that, for the pressure and temperature of the sample hydrocarbon-containing mixture, choked flow occurs. This is so that the flow rate of the sample hydrocarbon-containing mixture may be determined as a function of the molecular weight of the sample hydrocarbon-containing mixture.

The method comprises acquiring a measured value relatable to the residual $O_2$ concentration in the product gas formed from the sample hydrocarbon-containing mixture.

The apparatus comprises a first sensor configured to acquire a measured value relatable to the residual $O_2$ concentration in the product gas formed from the sample hydrocarbon-containing mixture and for generating an electronic signal in response to acquiring the measured value relatable to the residual $O_2$ concentration in the product gas. The first sensor may be any suitable sensor for measuring $O_2$ concentration in gases, for example, a zirconia oxide cell. The first sensor provides an electronic output signal (a measured value) that is related to the residual $O_2$ concentration of the product gas.

The method comprises acquiring a measured value relatable to molecular weight of the sample hydrocarbon-containing mixture.

The apparatus comprises a second sensor configured to acquire a measured value relatable to the molecular weight of the sample hydrocarbon-containing mixture and for generating an electronic signal in response to acquiring the measured value relatable to the molecular weight of the sample hydrocarbon-containing mixture.

Since the specific gravity of a gas is directly related to the molecular weight of the gas, a measured value relatable to the specific gravity is also relatable to the molecular weight of the gas. The second sensor may be any suitable sensor for measuring the molecular weight of gases, for example, a densitometer using a vibrating element. Sensors for measuring molecular weight of gases are well-known.

The method may comprise acquiring a measured value relatable to a hydrogen concentration of the sample hydrocarbon-containing mixture.

The apparatus may comprise a third sensor configured to acquire a measured value relatable to a hydrogen concentration of the sample hydrocarbon-containing mixture and for generating an electronic signal in response to acquiring the measured value relatable to the hydrogen concentration of the sample hydrocarbon-containing mixture. The third sensor may be any suitable sensor for measuring the $H_2$ concentration in the sample hydrocarbon-containing mixture, for example, a HY-OPTIMA™ sensor available from H2scan.

The method comprises determining a heating value of the sample hydrocarbon-containing mixture from the measured value relatable to the residual $O_2$ concentration in the product gas, the measured value relatable to molecular weight of the sample hydrocarbon-containing mixture, and, if acquired, the measured value relatable to a hydrogen concentration of the sample hydrocarbon-containing mixture

The apparatus comprises a computing device operatively connected to the first sensor, the second sensor, and the third sensor, if present, to receive electronic signals from the first sensor, the second sensor, and the third sensor, if present. The computing device is configured to calculate the heating value of the sample hydrocarbon-containing mixture from the measured value relatable to the residual $O_2$ concentration in the product gas and the measured value relatable to the molecular weight of the sample hydrocarbon-containing mixture. If the hydrogen concentration of the sample hydrocarbon-containing mixture is acquired, the computing device may also be configured to calculate the heating value of the sample hydrocarbon-containing mixture from the measured value relatable to a hydrogen concentration of the sample hydrocarbon-containing mixture. The computing device may be any suitable device capable of receiving electronic signals from the sensors and calculating heating values.

The sample hydrocarbon-containing mixture may contain hydrocarbon species and non-hydrocarbon species. The effect of hydrocarbon species and non-hydrocarbon species on the magnitude of the heating value is important. Some non-hydrocarbon species, such as $N_2$ and $CO_2$ contribute nothing to the magnitude of the heating value. The effect hydrocarbon species and non-hydrocarbon species on heating value can be determined using one or more model equations where mixture properties are determined from additive contributions from at least a first group of components (e.g. hydrocarbon components) and a second group of components (e.g. non-hydrocarbon components).

This approach using model equations is described in U.S. Pat. No. 7,871,826, incorporated herein by reference.

The heating value for the sample mixture, $HV$, may be written in terms of the contribution from a first group of components (generally hydrocarbon components) and a second group of components (generally non-hydrocarbon components):

$$HV = HV_1 \times Y + HV_2 \times (1 - Y) \qquad (1)$$

where $HV_1$ is the contribution to the mixture heating value, $HV$, by the group 1 components, $HV_2$ is the contribution to the mixture heating value by the group 2 components, and $Y$ is the mole fraction of the group 1 components.

The approach requires the measurement of two properties of the sample mixture for example the combustion oxidant requirement (COR) (or equivalently the combustion air requirement, CAR) and molecular weight.

The combustion oxidant requirement for the mixture can be written in terms of the contribution from the group 1 components and the contribution from the group 2 components:

$$COR = COR_1 \times Y + COR_2 \times (1 - Y) \qquad (2)$$

The molecular weight for the mixture can be written in terms of the contribution from the group 1 components and the contribution from the group 2 components:

$$MW = MW_1 \times Y + MW_2 \times (1 - Y) \qquad (3)$$

The inventors have found that the combustion oxygen requirement, COR, for the group 1 components is essentially linearly related to the heating value, HV, and can be correlated from data as

$$COR_1 = a_1 HV_1 + b_1. \qquad (4)$$

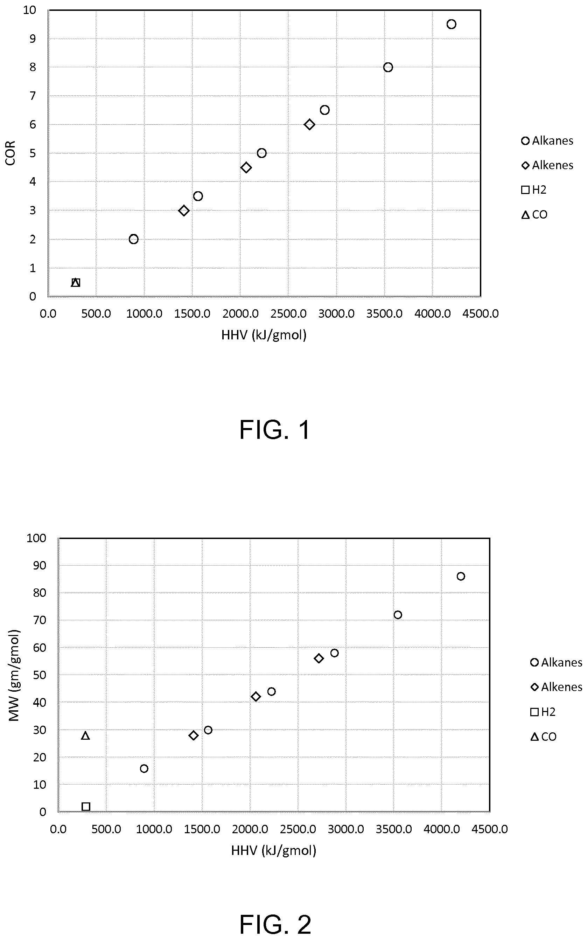

FIG. 1 shows a plot of COR as a function of heating value for alkanes and alkenes.

Likewise the molecular weight of the group 1 components is essentially linearly related to the heating value, HV, and can be correlated from data as

$$MW_1 = a_2 HV_1 + b_2. \qquad (5)$$

FIG. 2 shows a plot of molecular weight as a function of heating value for alkanes and alkenes.

Frequently some information about the sample mixture is known and some additional approximations can be made. For example, if the non-hydrocarbon species are $CO_2$ and $N_2$, $COR_2$ is zero. Further the molecular weight of $CO_2$ is 44 and the molecular weight of $N_2$ is 28, so an average value can be used without introducing too much error. Also the heating value for the second group, $HV_2$, maybe zero or a constant.

As a result, there are an equal number of equations as unknowns, and the heating value, HV, can be determined from the measurement or determination of two properties combustion oxidant requirement and molecular weight of the sample mixture.

These equations can be further modified to include the measurement of the hydrogen concentration. It can be further specified that:

$$HV_2 = y_{H_2} \times HV_{H_2} + (1 - Y - y_{H_2}) \times HV_{2-H_2} \qquad (6)$$

where $y_{H_2}$ is the mole fraction of $H_2$, $HV_{H_2}$ is the heating value of $H_2$, and $HU_{2-H_2}$ is the heating value of the group 2 components excluding $H_2$. Generally, $HV_{2-H_2}$ will be 0.

It can also be further specified that:

$$COR_2(1 - Y) = y_{H_2} \times COR_{H_2} + (1 - Y - y_{H_2}) \times COR_{2-H_2} \qquad (7)$$

where $COR_{H_2}$ is the combustion oxidant requirement for $H_2$, and $COR_{2-H_2}$ is the combustion oxidant requirement of the group 2 components excluding $H_2$. Generally $COR_{2-H_2}$ will be zero.

It can also be further specified that:

$$MW_2 \times (1 - Y) = y_{H_2} \times MW_{H_2} + (1 - Y - y_{H_2}) \times MW_{2-H_2} \qquad (8)$$

where $MW_{H_2}$ is the molecular weight of $H_2$, and $MW_{2-H_2}$ is the molecular weight of the group 2 components excluding $H_2$. Generally the group 2 components other than $H_2$ include $N_2$ and $CO_2$ and can be approximated using a constant value.

The computing device may be configured to calculate the heating value using a mathematical relationship derived from one or more model equations such as described above.

It is shown below how the combustion oxidant requirement can be determined from the residual $O_2$ concentration in the product gas.

The computing device may be configured to calculate the heating value of the sample hydrocarbon-containing mixture from a combustion oxygen requirement index. As used herein, "combustion oxygen requirement index" is the generic term to describe various indices for the stoichiometric oxidant gas/fuel ratio divided by specific gravity to a power of 0.4 to 0.6, preferably 0.5 (i.e. square root of specific gravity), or normalized molecular weight to a power of 0.4 to 0.6, preferably 0.5 (i.e. the square root of normalize molecular weight). A common example that is frequently used is the combustion air requirement index (CARI). The combustion air requirement index is the stoichiometric air-fuel ratio of a gas divided by the square root of the specific gravity of the gas.

The combustion oxygen requirement index may be determined using a correlation of the combustion oxygen requirement index as a function of the residual $O_2$ concentration and the measured value relatable to the residual $O_2$ concentration in the product gas formed from the sample hydrocarbon-containing mixture as input to the correlation.

The correlation may be determined analytically as described below.

First consider a case where the oxidant molar flow rate and the sample molar flow rate are constant and where the oxidant is air. Let A be a constant representing the molar flow rate of air. Let F be a constant representing the molar flow rate of the sample. Let COR (combustion oxidant ratio) be the stoichiometric moles of $O_2$ to react the sample to form $CO_2$ and $H_2O$. Then, $CAR$ (combustion air ratio) is the stoichiometric moles of air to react the sample to form $CO_2$ and $H_2O$.

The molar flow rate of $O_2$ that enters with air is $A*0.2095$. The molar flow rate of $N_2$ that enters with air is $A*(1-0.2095)$.

Alkanes and $H_2$ have the general molecular formula, $C_nH_{2n+2}$, where n=0 for $H_2$, n=1 for $CH_4$, etc. The combustion oxygen requirement (COR) for alkanes is COR=(3*n+1)/2.

Alkenes have the general molecular formula, $C_nH_{2n}$, where n=2 for $C_2H_4$, n=3 for $C_3H_6$, etc. The combustion oxygen requirement (COR) for alkenes is COR=(3*n)/2.

The molar rate of reaction for $O_2$ is $M_{O2, reacted} = F*COR$. The molar rate of unreacted $O_2$ passing to through to the product gas is:

$$M_{O2, unreacted} = 0.2095^*A\text{-}F^*COR. \qquad (9)$$

Equation 1 shows that for the case where A is constant and F is constant, the molar rate of unreacted $O_2$ passing to the product gas is linearly related to COR. This is an exact linear relationship. This equation can be rewritten as:

$$COR = (0.2095^*A - M_{O2, unreacted})/F. \qquad (9a)$$

This equation is basically a definition for the stoichiometric $O_2$ requirement or moles of $O_2$ required for complete reaction per mole of the sample.

The moles of combustion product gases, $CO_2$ and $H_2O$, can be expressed as a function of COR for alkanes and alkenes. For alkanes, the molar flow rate of product gases formed are $F*(4*COR+1)/3$. For alkenes, the molar flow rate of product gases formed are $F*(4*COR)/3$.

The total molar flow rate of product gases, P, produced by complete reaction of the sample is $P=A+F*(1*COR+1)/3$. The residual $O_2$ concentration $[O_2]$ is the moles of $O_2$ that are unreacted divided by the total moles of product gases and for alkanes can be expressed:

$$[O_2]=(0.2095*A-F*COR)/(A+F*(COR+1/3)). \qquad (10a)$$

For alkenes,

$$[O_2]=(0.2095*A-F*COR)/(A+F*(COR/3)). \qquad (10b)$$

The denominator in equations 10a and 10b can be shown to be approximately constant and do not vary too much for alkanes versus alkenes.

The molar flow rate, F, of the sample is given a basis flow rate of 1 mole/s. For a sample having only hexane (n=6), the value of A needs to be at least 50 moles/s in order to have sufficient $O_2$ to completely react the sample and have residual $O_2$.

For $H_2$ and alkanes ranging from C1 to C6, n ranges from 0 to 6. Correspondingly, COR ranges from 0.5 to 9.5. The term $F*(COR+1)/3$ ranges from (0.5+1)/3 to (9.5+1)/3, i.e. ranges from 0.5 to 3.5.

For alkenes ranging from C2 to C5, n ranges from 2 to 5. Correspondingly COR ranges from 3 to 7.5. The term $F*(COR)/3$ ranges from 3/3, to 7.5/3, i.e. from 1 to 2.5.

For alkanes, the denominator $(A+F*(COR+1)/3)$ ranges between 50.5 to 53.5. For alkenes, the denominator $(A+F*(COR)/3)$ ranges between 51 to 52.5. The denominator in equations 2a and 2b can be approximated as a constant average value without introducing too much error.

Consequently, the term (COR+1) for alkanes and (COR) in the denominator can be approximated using a constant, K.

Equations 10a and 10b can be rewritten:

$$[O_2]=(0.2095*A-F*COR)/(A+F*(K/3)).$$

Rearranging and solving for COR results in:

$$COR = \frac{0.2095A}{F} - \left(\frac{A}{F} + \frac{K}{3}\right)[O_2]. \qquad (11)$$

Since the molar flow rate of air, A, and the molar flow rate of the sample, F, are maintained constant, equation 11 shows a linear relationship between COR and the oxygen concentration.

If the range of compositions of the sample mixtures is somewhat known, the average value of K can be approximated.

Equation 11 can be easily rewritten in terms of CAR if desired.

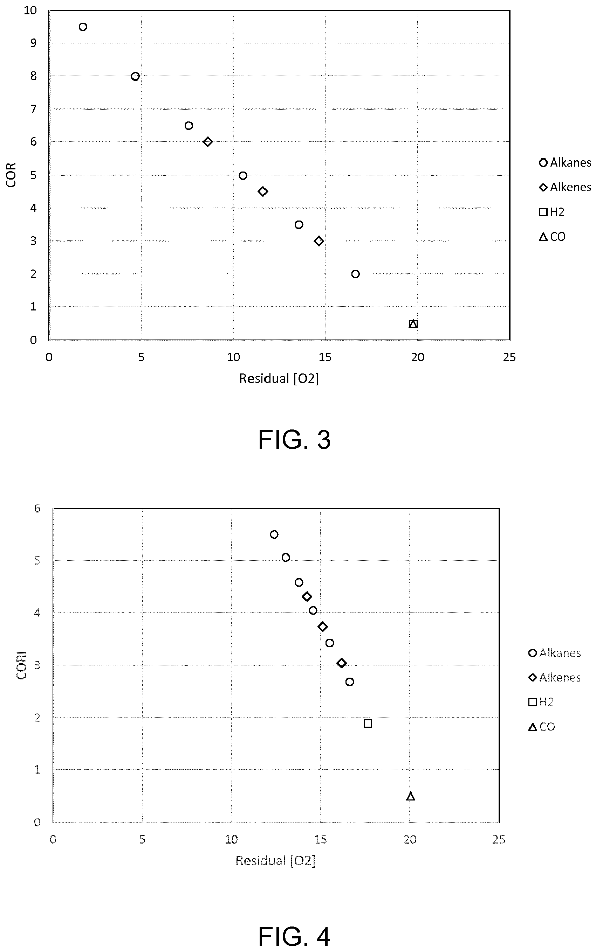

A plot of COR as a function of residual $O_2$ concentration is plotted in FIG. 3 for $H_2$, alkanes ranging from C1 to C6, alkenes ranging from C2 to C5, and CO. A near perfect linear relationship between COR and residual $O_2$ concentration is revealed.

The slow and intercept of equation 11 is explicitly expressed in terms of A, F, and K. When the values of A or F are changed, one can predict how the slope and intercept will change. If the values of A and F are fixed, then one only needs to run one calibration gas through the system as a means of checking and confirming the air and fuel ratios. As long as the ratio, A/F remains fixed, one can determine the curve from one point, i.e. the known COR value and the measured $O_2$ value.

The analysis above requires that the molar flow rate of air and the sample are constant. However, the molar flow rate through an orifice is known to change depending on the molecular weight of the sample. Since the molecular weight of air will remain unchanged, this requirement is satisfied. However, the molecular weight of the sample may change resulting in a variation in the molar flow rate of the sample.

Again the temperature and the pressure of the air and the sample are maintained constant and the flow is choked through the orifice.

The critical molar flow rate through an orifice can be shown to vary as $\dfrac{1}{\sqrt{MW}}$, where *MW* is the molecular weight. From Perry's Chemical Engineers' Handbook, 6th Edition, Perry and Green (ed.), 1984, equation 5-21 shows the relationship between the maximum-weight flow rate for a perfect gas as varying as $\sqrt{MW}$. Dividing through by *MW* to solve for the molar flow rate, the molar flow rate then varies as $\dfrac{1}{\sqrt{MW}}$.

Then to account for the molar flow rate of the sample varying with changes in molecular weight, F equals some constant divided by the square root of the molecular weight, $F = \dfrac{k}{\sqrt{MW}}$.

Substituting this into equation 9

$$M_{O2,\ unreacted} = 0.2095 \times A - \frac{k}{\sqrt{MW}} \times COR. \qquad (12)$$

Equation 13 shows a linear relationship between the combustion oxygen requirement index $\left( CORI = \dfrac{COR}{\sqrt{MW}} \right)$ and the moles of unreacted $O_2$.

Substituting $F = \dfrac{k}{\sqrt{MW}}$ into equation 12 and solving for *CORI:*

$$CORI = \frac{COR}{\sqrt{MW}} = \frac{0.2095A}{k} - \left( \frac{A}{k} + \frac{K}{3\sqrt{MW_{average}}} \right) [O_2]. \qquad (13)$$

A plot of *CORI* as a function of residual $O_2$ concentration is plotted in FIG. 4 for $H_2$, alkanes ranging from C1 to C6, alkenes ranging from C2 to C5, and CO. A near perfect linear relationship between *CORI* and residual $O_2$ concentration is revealed. The relationship between *CARI* and residual $O_2$ concentration can be readily determined from the oxygen concentration in air.

This analysis shows that the combustion oxygen requirement index, *CORI,* can be correlated with the residual oxygen concentration of the product mixture. COR can be calculated from *CORI* and the measured molecular weight.

Then the combustion oxidant requirement index, CORI, can be determined as a function of the residual $O_2$ concentration as shown in FIG. 4 and the combustion oxidant requirement, COR, can be calculated from the molecular weight and CORI.

From COR and molecular weight, the equations above can be solved to determine the heating value of the sample mixture.

The accuracy of the apparatus and the method may be improved using one or more calibration gases. The one or more calibration gases may be used prior to the sample hydrocarbon-containing mixture.

The method may further comprise reacting a calibration gas with the oxidant gas. The calibration gas may have a concentration of a single hydrocarbon component greater than 99 mole % or greater than 99.9 mole %. The calibration gas and the oxidant gas provided in a ratio to form a product gas having residual $O_2$ concentration. The calibration gas may be methane.

The calibration gas may be passed through the second orifice under choked flow conditions and into the reaction chamber for reacting the calibration gas with the oxidant gas in the reaction chamber. The calibration gas may be passed through the second orifice severally from the sample hydrocarbon-containing mixture. The calibration gas may be passed through the second orifice contemporaneously with passing the oxidant through the first orifice.

The method may further comprise acquiring a measured value relatable to the residual $O_2$ concentration in the product gas formed from the calibration gas, acquiring a measured value relatable to molecular weight of the calibration gas, and calibrating the correlation of the combustion oxygen requirement index as a function of the residual $O_2$ concentration using the measured value relatable to the residual $O_2$ concentration in the product gas formed from the calibration gas and the measured value relatable to the molecular weight of the calibration gas.

The apparatus may further comprise a source of a calibration gas, the calibration gas having a concentration of a single hydrocarbon component greater than 99 mole % or greater than 99.9 mole %. The reaction chamber is configured to selectively receive the calibration gas from the source of the calibration gas and to receive the oxidant gas. The reaction chamber may be operatively arranged to receive the calibration gas after the calibration gas is passed through the second orifice. The reaction chamber configured to receive the calibration gas severally (i.e. each by itself) from the sample hydrocarbon-containing mixture. The reaction chamber configured to discharge a product gas formed from the calibration gas, the product gas formed from the calibration gas having a residual $O_2$ concentration.

When the calibration gas is used, the the first sensor is configured to acquire a measured value relatable to the residual $O_2$ concentration in the product gas formed from the calibration gas and for generating an electronic signal in response thereto.

When the calibration gas is used, the second sensor is configured to acquire a measured value relatable to the molecular weight of the calibration gas and for generating an electronic signal in response thereto.

When the calibration gas is used, the computing device is configured to calculate the heating value of the sample hydrocarbon-containing mixture from the residual $O_2$ concentration in the product gas formed from the calibration gas and the measured value relatable to the molecular weight of the calibration gas.

The method may further comprise reacting a second calibration gas with the oxidant gas. The second calibration gas may have a concentration of a single hydrocarbon component greater than 99 mole % or greater than 99.9 mole %. The hydrocarbon component in the second calibration gas is different than the hydrocarbon component in the calibration gas. The second calibration gas and the oxidant gas provided in a ratio to form a product gas having residual $O_2$ concentration. The calibration gas may be ethane, propane, butane, pentane or hexane.

The second calibration gas may be passed through the second orifice under choked flow conditions and into the reaction chamber for reacting the second calibration gas with the oxidant gas in the reaction chamber. The second calibration gas may be passed through the second orifice severally from the sample hydrocarbon-containing mixture and severally from the calibration gas. The second calibration gas may be passed through the second orifice contemporaneously with passing the oxidant through the first orifice.

The method may further comprise acquiring a measured value relatable to the residual $O_2$ concentration in the product gas formed from the second calibration gas, acquiring a measured value relatable to molecular weight of the second calibration gas, and calibrating the correlation of the combustion oxygen requirement index as a function of the residual $O_2$ concentration using the measured value relatable to the residual $O_2$ concentration in the product gas formed from the second calibration gas and the measured value relatable to the molecular weight of the second calibration gas.

The apparatus may further comprise a source of a second calibration gas, the second calibration gas having a concentration of a single hydrocarbon component greater than 99 mole % or greater than 99.9 mole %. The reaction chamber is configured to selectively receive the second calibration gas from the source of the second calibration gas and to receive the oxidant gas. The reaction chamber may be operatively arranged to receive the second calibration gas after the second calibration gas is passed through the second orifice. The reaction chamber configured to receive the second calibration gas severally (i.e. each by itself) from the sample hydrocarbon-containing mixture and the calibration gas. The reaction chamber configured to discharge a product gas formed from the second calibration gas, the product gas formed from the second calibration gas having a residual $O_2$ concentration.

When the second calibration gas is used, the the first sensor is configured to acquire a measured value relatable to the residual $O_2$ concentration in the product gas formed from the second calibration gas and for generating an electronic signal in response thereto.

When the second calibration gas is used, the second sensor is configured to acquire a measured value relatable to the molecular weight of the second calibration gas and for generating an electronic signal in response thereto.

When the second calibration gas is used, the computing device is configured to calculate the heating value of the sample hydrocarbon-containing mixture from the residual $O_2$ concentration in the product gas formed from the second calibration gas and the measured value relatable to the molecular weight of the second calibration gas.

The calibration gases may be used to test the accuracy of the sensors and the sensor response may be modified accordingly or the algorithm for determining the heating value may compensate for drift in the sensor response.

The method may further comprise calculating a carbon content value of the sample hydrocarbon-containing mixture using the combustion oxygen requirement index of the sample hydrocarbon-containing mixture and the measured value relatable to the molecular weight of the sample hydrocarbon-containing mixture in a carbon content correlation.

The method may further comprise calculating a carbon content value of the sample hydrocarbon-containing mixture using the heating value of the sample hydrocarbon-containing mixture and the measured value relatable to the molecular weight of the sample hydrocarbon-containing mixture in a carbon content correlation.

Calculation of the carbon content value is described in U.S. Pat. No. 7,871,826, incorporated herein by reference.

EXAMPLE

The heating value for a hypothetical mixture is determined.

The calculations that are carried out for the case where the molecular weight of the sample mixture is measured, the residual $O_2$ concentration after combusting the sample mixture with a given air-to-fuel mixture is measured and the measurement is correlated to the combustion air requirement (CAR), and the $H_2$ concentration of the sample mixture is measured.

The method described below for the example uses the model equations developed and assumptions made herein but using the combustion air requirement index (CAR) instead of the combustion oxidant requirement index. The higher heating value, HHV will be used for the heating value. Equations 1 through 8 are summarized below:

$$HHV = HHV_1 \times Y + HHV_2 \times (1-Y) \qquad (E1)$$

$$CAR = CAR_1 \times Y + CAR_2 \times (1-Y) \qquad (E2)$$

$$MW = MW_1 \times Y + MW_2 \times (1-Y) \qquad (E3)$$

$$CAR_1 = a_1 HHV_1 + b_1. \qquad (E4)$$

$$MW_1 = a_2 HHV_1 + b_2. \qquad (E5)$$

$$HHV_2 = y_{H_2} \times HHV_{H_2} + (1 - Y - y_{H_2}) \times HHV_{2-H_2} \qquad (E6)$$

$$CAR_2(1-Y) = y_{H_2} \times CAR_{H_2} + (1 - Y - y_{H_2}) \times CAR_{2-H_2} \qquad (E7)$$

$$MW_2 = y_{H_2} \times MW_{H_2} + (1 - Y - y_{H_2}) \times MW_{2-H_2} \qquad (E8)$$

In this example, the group 1 components include the paraffins and the olefins. The group 2 components include $H_2$, $CO_2$, and $N_2$.

In this example, we consider the case where the $H_2$ concentration is measured or otherwise known or approximated.

In this case $HV_{2-H2}=0$ and $CAR_{2-H2}=0$.

Substituting equation E6 into equation E1 gives

$$HHV = HHV_1 \times Y + y_{H_2} \times HHV_{H_2} + (1 - Y - y_{H_2}) \times HHV_{2-H_2} \qquad (E1')$$

In this case, the last term is zero and $HV_{H2}$ is a known value of 286.1 kJ/mol.

Substituting equation E7 into equation E2 gives:

$$CAR = CAR_1 \times Y + y_{H_2} \times CAR_{H_2} + (1 - Y - y_{H_2}) \times CAR_{2-H_2} \qquad (E2')$$

In this case, the last term is zero and $CAR_{H2}$ is a known value of 0.5 mole $O_2$ per mole of $H_2$.

Substituting equation E8 into equation E3 gives:

$$MW = MW_1 \times Y + MW_{H_2} \times y_{H_2} + MW_{2-H_2} \times \left(1 - Y - y_{H_2}\right) \qquad \text{(E3')}$$

In this case, $MW_{H2}$ is a known value of 2.016 g/mole and $MW_{2-H2}$ is the expected molecular weight for the blend of $CO_2$ and $N_2$.

The equations that need to be solved are E1', E2', E3', E4, and E5.

Equations E4 and E5 can be substituted into equations E2' and E3' to obtain two equations with two unknown quantities, $HHV_1$ and Y, with the result:

$$CAR = \left(a_1 \times HHV_1 + b_1\right) \times Y + y_{H_2} \times CAR_{H_2} \qquad \text{(E2'')}$$

$$MW = \left(a_2 \times HHV_2 + b_2\right) \times Y + y_{H_2} MW_{H_2} + MW_{2-H_2} \times \left(1 - Y - y_{H_2}\right) \qquad \text{(E3'')}$$

In this example, $MW_{2\text{-}H2} = MW_{N2} = 28.01$.

This specific example is carried out for a sample mixture having the following composition:

$N_2$ = 10 mole %
$C_2H_4$ = 2 mole %
H2 = 20 mole %
$CH_4$ = mole 60%
$C_2H_6$ = mole 5%
$C_3H_8$ = mole 3%

The following bulk properties were determined:

1st Bulk Property, $P_1$ = MW = 16.218

MW is obtained directly from the densitometer measurement. The density is correlated to the period of oscillation of the vibrating element. The density is measured at a fixed T & P.

In the present method, the densitometer can be calibrated using 2 pure calibration gases. For example, methane and ethane can be used as calibration gases.

2nd Bulk Property, $P_2$ = CAR

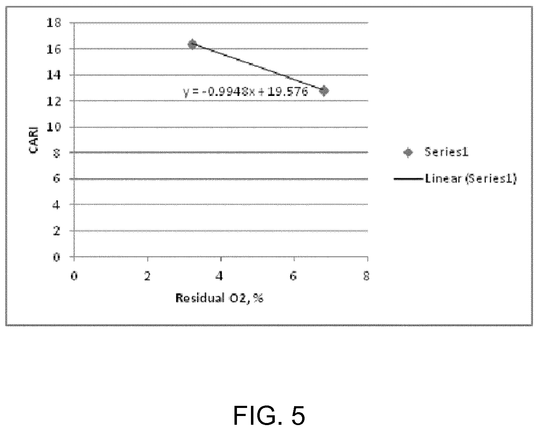

The combustion air requirement index (CARI) is obtained from a correlation relating the measured residual $O_2$ concentration to the CARI. CARI is defined in this example as: CARI = CAR/(SG)$^{0.5}$. CAR is the Combustion Air Requirement or the stoichiometric air-to-fuel ratio. Specific Gravity (SG) = $MW/MW_{air}$ = MW/28.95. The correlation is shown in FIG. 5.

The residual $O_2$ concentration is measured by combusting a mixture of air and fuel at a known air-to-fuel molar ratio. With fixed air and sample mixture metering orifice sizes, this ratio is controlled by fixing the temperature and pressure of the air and fuel streams supplied to their respective metering orifices.

The air-to-fuel molar ratio (for a given set of metering orifices and given T & P) is given by:

$$\text{Air/fuel} = 14.6 \times (MW/16.043)^{1/2} = 14.679 \text{ moles air/mole sample mixture}$$

This equation accounts for the change in the sample mixture flow rate due to a change in the MW of the sample mixture.

A 2-point calibration using methane and ethane is shown in FIG. 5.

How the calibration curve is used to get CAR for the test mixture is described below:

Combustion of a mixture of the fuel sample with dry air at this air-to-fuel ratio produces combustion products having 8.8928% $O_2$.

Correlation relating CARI to Residual O2:

$$CARI\_Pred = -0.9948 \cdot O2 + 19.576 = 10.7294$$

For comparison purposes, the actual value of CARI (calculated based on the fuel composition) is CARI_comp = 10.7459.

This correlation relating CARI to residual $O_2$ concentration, which can be achieved with pure cal gases, was found to be very accurate across a broad range of composition. This includes extremely wide ranges of ratios of $H_2$ : olefins : paraffins.

Therefore, CAR = $10.7294 \cdot (16.218/28.95)^{0.5}$ = 8.0305

The values of $a_1$, $b_1$, $a_2$, and $b_2$ are obtained as follows.

First, obtain values of slopes and intercepts for CAR as a function of HHV for paraffins and olefins. The result for paraffins is $a_1$=0.0108 and $b_1$=-0.1773 and for olefins is $a_1$=0.011 and $b_1$=-1.1266.

In this example, the ratio of olefins to (paraffins+olefins) is

$$\frac{y_{olefin}}{\left(y_{olefin} + y_{paraffin}\right)} = \frac{0.02}{\left(0.02 + 0.6 + 0.05 + 0.03\right)} = 0.028$$

For the expected blend of paraffins and olefins, the slopes and intercepts are obtained by proportionally weighting the values for the slopes and intercepts for the paraffins and olefins. For example, a1 = 0.0108x(1-0.028)+0.011x(0.028)=0.01086. Using the same approach, $b_1$=-0.2039.

The slope and intercepts for paraffins (alkanes) and olefins (alkenes) were obtained by fitting a straight line to the corresponding pur component data as illustrated in FIG. 1. Since air is assumed to contain 20.95 mole % $O_2$, the value of CAR and COR are related by CAR=COR/0.2095.

Likewise, the slopes and intercepts are obtained for molecular weight, MW, as a function of higher heating value, HHV. The slopes and intercepts were obtained by fitting a straight line tohte corresponding pure component data from FIG. 2. The result for paraffins is $a_1$=0.0213 and $b_1$=-3.0069 and for olefins is $a_1$=0.0215 and $b_1$=-2.2072. The result for the blend is $a_2$=0.0213, and $b_2$=-2.9845

Equations E2" and E3" are solved for Y and $HHV_1$ with the result Y=0.702 and $HHV_1$=1014.1 kJ/mole.

Using these values into equation E1' gives the result HHV=769.5 kJ/mole. This compares well with the value of HHV determined from the composition, which is 764.8kJ/mole.

The relative error in the higher heating value determined by this method versus the higher heating value determined from the composition is +0.6%.

**Claims**

1. An apparatus for determining a heating value of a sample hydrocarbon-containing mixture having hydrocarbons and non-hydrocarbons as mixture components, the apparatus comprising:

   a reaction chamber configured to selectively receive the sample hydrocarbon-containing mixture and an oxidant gas and discharge a product gas having a residual $O_2$ concentration where the product gas is formed from the sample hydrocarbon-containing mixture and the oxidant gas;
   a first sensor configured to acquire a measured value relatable to the residual $O_2$ concentration in the product gas and for generating an electronic signal in response thereto;
   a second sensor configured to acquire a measured value relatable to the molecular weight of the sample hydrocarbon-containing mixture and for generating an electronic signal in response thereto; and
   a computing device operatively connected to the first sensor and the second sensor to receive the electronic signals from the first sensor and the second sensor, wherein the computing device is configured to calculate the heating value of the sample hydrocarbon-containing mixture from a combustion oxygen requirement index and the measured value relatable to molecular weight, wherein the combustion oxygen requirement index is determined using a correlation of the combustion oxygen requirement index as a function of the residual $O_2$ concentration and using the measured value relatable to the residual $O_2$ concentration in the product gas as input to the correlation.

2. The apparatus of claim 1 further comprising:

a third sensor configured to acquire a measured value relatable to a hydrogen concentration of the sample hydrocarbon-containing mixture and for generating an electronic signal in response thereto;

wherein the computing device is also operatively connected to the third sensor to receive the electronic signals from the third sensor, wherein the computing device is configured to calculate the heating value of the sample hydrocarbon-containing mixture from the measured value relatable to the hydrogen concentration of the sample hydrocarbon-containing mixture in addition to the combustion oxygen requirement index and the measured value relatable to the molecular weight.

3. The apparatus of claim 1 or 2 wherein the computing device is configured to calculate the heating value of the sample hydrocarbon-containing mixture using a mathematical relationship derived from one or more model equations where mixture properties are determined from additive contributions from at least a first group of components and a second group of components.

4. The apparatus of any one of claims 1 to 3 further comprising:

a source of a calibration gas, the calibration gas having a concentration of a single hydrocarbon component greater than 99 mole % or greater than 99.9 mole %,

wherein the reaction chamber is configured to selectively receive the calibration gas from the source of the calibration gas and to receive the oxidant gas, the reaction chamber configured to receive the calibration gas severally from the sample hydrocarbon-containing mixture, the reaction chamber configured to discharge a product gas formed from the calibration gas, the product gas formed from the calibration gas having a residual $O_2$ concentration;

wherein the first sensor is configured to acquire a measured value relatable to the residual $O_2$ concentration in the product gas formed from the calibration gas and for generating an electronic signal in response thereto;

wherein the second sensor is configured to acquire a measured value relatable to the molecular weight of the calibration gas and for generating an electronic signal in response thereto; and

wherein the computing device is configured to calibrate the correlation of the combustion oxygen requirement index as a function of the residual $O_2$ concentration using the measured value relatable to the residual $O_2$ concentration in the product gas formed from the calibration gas and the measured value relatable to the molecular weight of the calibration gas.

5. The apparatus of claim 4 further comprising:

a source of a second calibration gas, the second calibration gas having a concentration of a single hydrocarbon component greater than 99 mole % or greater than 99.9 mole %, where the hydrocarbon component in the second calibration gas is different than the hydrocarbon component in the calibration gas;

wherein the reaction chamber is configured to selectively receive the second calibration gas from the source of the second calibration gas and to receive the oxidant gas, the reaction chamber configured to receive the second calibration gas severally from the sample hydrocarbon-containing mixture and severally from the calibration gas, the reaction chamber configured to discharge a product gas formed from the second calibration gas, the product gas formed from the second calibration gas having a residual $O_2$ concentration;

wherein the first sensor is configured to acquire a measured value relatable to the residual $O_2$ concentration in the product gas formed from the second calibration gas and for generating an electronic signal in response thereto;

wherein the second sensor is configured to acquire a measured value relatable to the molecular weight of the second calibration gas and for generating an electronic signal in response thereto; and

wherein the computing device is configured to calibrate the correlation of the combustion oxygen requirement index as a function of the residual $O_2$ concentration using the measured value relatable to the residual $O_2$ concentration in the product gas formed from the second calibration gas and the measured value relatable to the molecular weight of the second calibration gas.

6. A method for determining a heating value of a sample hydrocarbon-containing mixture having hydrocarbons and non-hydrocarbons as mixture components, the method comprising:

(a) reacting the sample hydrocarbon-containing mixture with an oxidant gas, the sample hydrocarbon-containing mixture and the oxidant gas provided in a ratio to form a product gas having a residual $O_2$ concentration;

(b) acquiring a measured value relatable to the residual $O_2$ concentration in the product gas;

(c) acquiring a measured value relatable to molecular weight of the sample hydrocarbon-containing mixture; and

(d) determining a heating value of the sample hydrocarbon-containing mixture from a combustion oxygen requirement index and the measured value relatable to molecular weight, wherein the combustion oxygen requirement index is determined using a correlation of the combustion oxygen requirement index as a function of the residual $O_2$ concentration and the measured value relatable to the residual $O_2$ concentration.

7. The method of claim 6 further comprising:

acquiring a measured value relatable to a hydrogen concentration of the sample hydrocarbon-containing mixture; wherein the heating value of the sample hydrocarbon-containing mixture is also determined from the measured value relatable to the hydrogen concentration of the sample hydrocarbon-containing mixture.

8. The method of claim 6 or 7 wherein the heating value of the sample hydrocarbon-containing mixture is determined using a mathematical relationship derived from one or more model equations where mixture properties are determined from additive contributions from at least a first group of components and a second group of components.

9. The method of any one of claims 6 to 8 wherein prior to steps (a) - (d), the method further comprises:

reacting a calibration gas with the oxidant gas, the calibration gas having a concentration of a single hydrocarbon component greater than 99 mole % or greater than 99.9 mole %, the calibration gas and the oxidant gas provided in a ratio to form a product gas having residual $O_2$ concentration; acquiring a measured value relatable to the residual $O_2$ concentration in the product gas formed from the calibration gas; acquiring a measured value relatable to molecular weight of the calibration gas; and calibrating the correlation of the combustion oxygen requirement index as a function of the residual $O_2$ concentration using the measured value relatable to the residual $O_2$ concentration in the product gas formed from the calibration gas and the measured value relatable to the molecular weight of the calibration gas.

10. The method of claim 9 wherein prior to steps (a) - (d), the method further comprises:

reacting a second calibration gas with the oxidant gas, the second calibration gas having a concentration of a single hydrocarbon component greater than 99 mole % or greater than 99.9 mole % where the hydrocarbon component in the second calibration gas is different than the hydrocarbon component in the calibration gas, the second calibration gas and the oxidant gas provided in a ratio to form a product gas having residual $O_2$ concentration; acquiring a measured value relatable to the residual $O_2$ concentration in the product gas formed from the second calibration gas; acquiring a measured value relatable to molecular weight of the second calibration gas; calibrating the correlation of the combustion oxygen requirement index as a function of the residual $O_2$ concentration using the measured value relatable to the residual $O_2$ concentration in the product gas formed from the second calibration gas and the measured value relatable to the molecular weight of the second calibration gas.

11. The method of any one of claims 6 to 10 further comprising:

calculating a carbon content value of the sample hydrocarbon-containing mixture using the combustion oxygen requirement index of the sample hydrocarbon-containing mixture and the measured value relatable to the molecular weight of the sample hydrocarbon-containing mixture in a carbon content correlation.

12. The method of any one of claims 6 to 11 further comprising:

calculating a carbon content value of the sample hydrocarbon-containing mixture using the heating value of the sample hydrocarbon-containing mixture and the measured value relatable to the molecular weight of the sample hydrocarbon-containing mixture in a carbon content correlation.

13. An apparatus for determining a heating value of a sample hydrocarbon-containing mixture having hydrocarbons and non-hydrocarbons as mixture components, the apparatus comprising:

a reaction chamber configured to selectively receive the sample hydrocarbon-containing mixture and an oxidant gas, and to discharge a product gas having residual $O_2$ concentration where the product gas is formed from the sample hydrocarbon-containing mixture and the oxidant gas;

a first sensor configured to acquire a measured value relatable to the residual $O_2$ concentration in the product gas and for generating an electronic signal in response thereto;

a second sensor configured to acquire a measured value relatable to the molecular weight of the sample hydrocarbon-containing mixture and for generating an electronic signal in response thereto;

a third sensor configured to acquire a measured value relatable to a hydrogen concentration of the sample hydrocarbon-containing mixture and for generating an electronic signal in response thereto;

a computing device operatively connected to the first sensor, second sensor, and the third sensor to receive the electronic signals from the first sensor, the second sensor, and the third sensor, wherein the computing device is configured to calculate the heating value of the sample hydrocarbon-containing mixture from the measured value relatable to the residual $O_2$ concentration in the product gas, the measured value relatable to the molecular weight of the sample hydrocarbon-containing mixture, and the measured value relatable to a hydrogen concentration of the sample hydrocarbon-containing mixture.

**14.** The apparatus of claim 13 further comprising:

a source of a calibration gas, the calibration gas having a concentration of a single hydrocarbon component greater than 99 mole % or greater than 99.9 mole %,

wherein the reaction chamber is configured to selectively receive the calibration gas from the source of the calibration gas and to receive the oxidant gas, the reaction chamber configured to receive the calibration gas severally from the sample hydrocarbon-containing mixture, the reaction chamber configured to discharge a product gas formed from the calibration gas, the product gas formed from the calibration gas having a residual $O_2$ concentration;

wherein the first sensor is configured to acquire a measured value relatable to the residual $O_2$ concentration in the product gas formed from the calibration gas and for generating an electronic signal in response thereto;

wherein the second sensor is configured to acquire a measured value relatable to the molecular weight of the calibration gas and for generating an electronic signal in response thereto; and

wherein the computing device is configured to calculate the heating value of the sample hydrocarbon-containing mixture from the residual $O_2$ concentration in the product gas formed from the calibration gas and the measured value relatable to the molecular weight of the calibration gas in addition to the measured value relatable to the residual $O_2$ concentration in the product gas formed from the sample hydrocarbon-containing mixture, the measured value relatable to the molecular weight of the sample hydrocarbon-containing mixture, and the measured value relatable to a hydrogen concentration of the sample hydrocarbon-containing mixture.

**15.** A method for determining a heating value of a sample hydrocarbon-containing mixture having hydrocarbons and non-hydrocarbons as mixture components, the method comprising:

(a) reacting the sample hydrocarbon-containing mixture with an oxidant gas, the sample hydrocarbon-containing mixture and the oxidant gas provided in a ratio to form a product gas having a residual $O_2$ concentration;

(b) acquiring a measured value relatable to the residual $O_2$ concentration in the product gas;

(c) acquiring a measured value relatable to molecular weight of the sample hydrocarbon-containing mixture;

(d) acquiring a measured value relatable to a hydrogen concentration of the sample hydrocarbon-containing mixture; and

(e) determining a heating value of the sample hydrocarbon-containing mixture from the measured value relatable to the residual $O_2$ concentration in the product gas, the measured value relatable to molecular weight of the sample hydrocarbon-containing mixture, and the measured value relatable to a hydrogen concentration of the sample hydrocarbon-containing mixture.

**16.** The method of claim 15 wherein prior to steps (a) - (e), the method further comprises:

reacting a calibration gas with the oxidant gas, the calibration gas having a concentration of a single hydrocarbon component greater than 99 mole % or greater than 99.9 mole %, the calibration gas and the oxidant gas provided in a ratio to form a product gas having residual $O_2$ concentration;

acquiring a measured value relatable to the residual $O_2$ concentration in the product gas formed from the calibration gas; and

acquiring a measured value relatable to molecular weight of the calibration gas;

wherein the heating value of the sample hydrocarbon-containing mixture is determined from the measured value relatable to the residual $O_2$ concentration in the product gas formed from the calibration gas, and the measured value relatable to molecular weight of the calibration gas in addition to the measured value relatable to the residual $O_2$ concentration in the product gas formed from the sample hydrocarbon-containing mixture, the measured value relatable to molecular weight of the sample hydrocarbon-containing mixture, and the measured value relatable to a hydrogen concentration of the sample hydrocarbon-containing mixture.

17. An apparatus for determining a heating value of a sample hydrocarbon-containing mixture having hydrocarbons and non-hydrocarbons as mixture components, the apparatus comprising:

a source of a calibration gas, the calibration gas having a concentration of a single hydrocarbon component greater than 99 mole % or greater than 99.9 mole %, a reaction chamber configured to selectively receive the sample hydrocarbon-containing mixture and an oxidant gas, and to discharge a product gas having residual $O_2$ concentration where the product gas is formed from the sample hydrocarbon-containing mixture and the oxidant gas, wherein the reaction chamber is configured to selectively receive the calibration gas from the source of the calibration gas and to receive the oxidant gas with the calibration gas, the reaction chamber configured to receive the calibration gas severally from the sample hydrocarbon-containing mixture, the reaction chamber configured to discharge a product gas formed from the calibration gas, the product gas formed from the calibration gas having a residual $O_2$ concentration;
a first sensor configured to acquire a measured value relatable to the residual $O_2$ concentration in the product gas formed from the sample hydrocarbon-containing mixture and for generating an electronic signal in response thereto, and the first sensor is configured to acquire a measured value relatable to the residual $O_2$ concentration in the product gas formed from the calibration gas and for generating an electronic signal in response thereto;
a second sensor configured to acquire a measured value relatable to the molecular weight of the sample hydrocarbon-containing mixture and for generating an electronic signal in response thereto, and the second sensor is configured to acquire a measured value relatable to the molecular weight of the calibration gas and for generating an electronic signal in response thereto;
a computing device operatively connected to the first sensor and the second sensor to receive the electronic signals from the first sensor and the second sensor, wherein the computing device is configured to calculate the heating value of the sample hydrocarbon-containing mixture from the residual $O_2$ concentration in the product gas formed from the calibration gas, the measured value relatable to the molecular weight of the calibration gas, the measured value relatable to the residual $O_2$ concentration in the product gas formed from the sample hydrocarbon-containing mixture, and the measured value relatable to the molecular weight of the sample hydrocarbon-containing mixture.

18. The apparatus of claim 17 further comprising:

a source of a second calibration gas, the second calibration gas having a concentration of a single hydrocarbon component greater than 99 mole % or greater than 99.9 mole %, where the hydrocarbon component in the second calibration gas is different than the hydrocarbon component in the calibration gas;
wherein the reaction chamber is configured to selectively receive the second calibration gas from the source of the second calibration gas and to receive the oxidant gas, the reaction chamber configured to receive the second calibration gas severally from the sample hydrocarbon-containing mixture and severally from the calibration gas, the reaction chamber configured to discharge a product gas formed from the second calibration gas, the product gas formed from the second calibration gas having a residual $O_2$ concentration;
wherein the first sensor is configured to acquire a measured value relatable to the residual $O_2$ concentration in the product gas formed from the second calibration gas and for generating an electronic signal in response thereto;
wherein the second sensor is configured to acquire a measured value relatable to the molecular weight of the second calibration gas and for generating an electronic signal in response thereto; and
wherein the computing device is configured to calculate the heating value of the sample hydrocarbon-containing mixture from the residual $O_2$ concentration in the product gas formed from the second calibration gas and the measured value relatable to the molecular weight of the second calibration gas in addition to the residual $O_2$ concentration in the product gas formed from the calibration gas, the measured value relatable to the molecular weight of the calibration gas, the measured value relatable to the residual $O_2$ concentration in the product gas formed from the sample hydrocarbon-containing mixture, and the measured value relatable to the molecular weight of the sample hydrocarbon-containing mixture.

19. A method for determining a heating value of a sample hydrocarbon-containing mixture having hydrocarbons and non-hydrocarbons as mixture components, the method comprising:

(a) reacting a calibration gas with the oxidant gas, the calibration gas having a concentration of a single hydrocarbon component greater than 99 mole % or greater than 99.9 mole %, the calibration gas and the oxidant gas provided in a ratio to form a product gas having residual $O_2$ concentration;

(b) acquiring a measured value relatable to the residual $O_2$ concentration in the product gas formed from the calibration gas; and

(c) acquiring a measured value relatable to molecular weight of the calibration gas;

(d) reacting the sample hydrocarbon-containing mixture with an oxidant gas, the sample hydrocarbon-containing mixture and the oxidant gas provided in a ratio to form a product gas having a residual $O_2$ concentration;

(e) acquiring a measured value relatable to the residual $O_2$ concentration in the product gas;

(f) acquiring a measured value relatable to molecular weight of the sample hydrocarbon-containing mixture; and

(g) determining a heating value of the sample hydrocarbon-containing mixture from the measured value relatable to the residual $O_2$ concentration in the product gas formed from the calibration gas, and the measured value relatable to molecular weight of the calibration gas, the measured value relatable to the residual $O_2$ concentration in the product gas formed from the sample hydrocarbon-containing mixture, and the measured value relatable to molecular weight of the sample hydrocarbon-containing mixture.

20. The method of claim 19 wherein prior to steps (d) - (g), the method further comprises:

reacting a second calibration gas with the oxidant gas, the second calibration gas having a concentration of a single hydrocarbon component greater than 99 mole % or greater than 99.9 mole % where the hydrocarbon component in the second calibration gas is different than the hydrocarbon component in the calibration gas, the second calibration gas and the oxidant gas provided in a ratio to form a product gas having residual $O_2$ concentration;

acquiring a measured value relatable to the residual $O_2$ concentration in the product gas formed from the second calibration gas;

acquiring a measured value relatable to molecular weight of the second calibration gas;

wherein the heating value of the sample hydrocarbon-containing mixture is determined from the measured value relatable to the residual $O_2$ concentration in the product gas formed from the second calibration gas, and the measured value relatable to molecular weight of the second calibration gas in addition to the measured value relatable to the residual $O_2$ concentration in the product gas formed from the calibration gas, the measured value relatable to the molecular weight of the calibration gas, the measured value relatable to the residual $O_2$ concentration in the product gas formed from the sample hydrocarbon-containing mixture, the measured value relatable to molecular weight of the sample hydrocarbon-containing mixture, and the measured value relatable to a hydrogen concentration of the sample hydrocarbon-containing mixture.

FIG. 1

FIG. 2

FIG. 3

FIG. 4

FIG. 5

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 16 19 5143

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 197 07 659 A1 (BETR FORSCH INST ANGEW FORSCH [DE]) 27 August 1998 (1998-08-27) | 1,3-6, 8-12 | INV. G01N33/22 |
| Y | * page 6, lines 12-54; figure 3 * | 2,7 | |
| A | EP 0 098 716 A1 (BABCOCK & WILCOX CO [US]) 18 January 1984 (1984-01-18) * abstract; figures * | 1-12 | |
| Y | US 2014/262836 A1 (LIU ZHIQIANG [CN] ET AL) 18 September 2014 (2014-09-18) * abstract; figure 1 * | 2,7 | |
| A | CN 2 886 570 Y (ZHANG DONGPING [CN]) 4 April 2007 (2007-04-04) * claim 1; figure * | 1-12 | |

TECHNICAL FIELDS
SEARCHED      (IPC)

G01N

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 1 March 2017 | Wilhelm-Shalganov, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04E07)

Europäisches
Patentamt

European
Patent Office

Office européen
des brevets

**INCOMPLETE SEARCH
SHEET C**

Application Number

EP 16 19 5143

Claim(s) completely searchable:
        1-12

Claim(s) not searched:
        13-20

Reason for the limitation of the search:

Claims 1, 6, 13, 15, 17 and 19 have been drafted as separate independent
claims. Under Article 84 in combination with Rule 43(2) EPC, an
application may contain more than one independent claim in a particular
category only if the subject-matter claimed falls within one or more of
the exceptional situations set out in paragraph (a), (b) or (c) of Rule
43(2) EPC. This is not the case in the present application.
In accordance with the applicant's request, the search has been
restricted to independent claims 1 and 6 and their respective dependent
claims.

## ANNEX TO THE EUROPEAN SEARCH REPORT
## ON EUROPEAN PATENT APPLICATION NO.

EP 16 19 5143

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2017

| Patent document cited in search report | | | Publication date | Patent family member(s) | | | Publication date |
|---|---|---|---|---|---|---|---|
| DE 19707659 | A1 | | 27-08-1998 | NONE | | | |
| EP 0098716 | A1 | | 18-01-1984 | AU | 558717 | B2 | 05-02-1987 |
| | | | | BR | 8303591 | A | 14-02-1984 |
| | | | | CA | 1183370 | A | 05-03-1985 |
| | | | | DE | 3370541 | D1 | 30-04-1987 |
| | | | | EP | 0098716 | A1 | 18-01-1984 |
| | | | | ES | 8404813 | A1 | 16-08-1984 |
| | | | | HK | 80487 | A | 06-11-1987 |
| | | | | IN | 161033 | B | 19-09-1987 |
| | | | | JP | H052059 | U | 14-01-1993 |
| | | | | JP | S5923239 | A | 06-02-1984 |
| | | | | MX | 153789 | A | 09-01-1987 |
| | | | | SG | 52287 | G | 28-08-1987 |
| | | | | US | 4433922 | A | 28-02-1984 |
| US 2014262836 | A1 | | 18-09-2014 | CN | 102539374 | A | 04-07-2012 |
| | | | | EP | 2796856 | A1 | 29-10-2014 |
| | | | | US | 2014262836 | A1 | 18-09-2014 |
| | | | | WO | 2013091399 | A1 | 27-06-2013 |
| CN 2886570 | Y | | 04-04-2007 | NONE | | | |

EPO FORM P0459

For more details about this annex : see Official Journal of the European Patent Office, No. 12/82

**REFERENCES CITED IN THE DESCRIPTION**

**Patent documents cited in the description**

- US 62245604 B

- US 7871826 B

**Non-patent literature cited in the description**

- Perry's Chemical Engineers' Handbook. 1984